# EUROPEAN PATENT SPECIFICATION

(11) **EP 1 809 316 B1**
(45) Date of publication and mention of the grant of the patent: **31.12.2008**
(21) Application number: 05811185.7
(22) Date of filing: 08.11.2005
(51) Int. Cl.: A61K 38/17, A61P 13/12

(54) **USE OF SURVIVIN TO TREAT KIDNEY FAILURE**
VERWENDUNG VON SURVIVIN ZUR BEHANDLUNG VON NIERENINSUFFIZIENZ
EMPLOI DE SURVIVINE DANS LE TRAITEMENT D`INSUFFISANCES RÉNALES

(30) Priority: 12.11.2004 EP 04105744
(43) Date of publication of application: 25.07.2007
(73) Proprietor: VIB vzw, 9052 Zwijnaarde (BE); Life Sciences Research Partners VZW., 3000 Leuven (BE)
(72) Inventor: CONWAY, Edward, B-3090 Overijse (BE)
(86) International application number: PCT/EP2005/055831
(87) International publication number: WO 2006/051075

(56) References cited:
- WO-A-98/22589
- DOHI, TAKEHIKO ET AL: "An IAP-IAP Complex Inhibits Apoptosis" JOURNAL OF BIOLOGICAL CHEMISTRY , 279(33), 34087-34090 CODEN: JBCHA3; ISSN: 0021-9258, 13 August 2004 (2004-08-13), XP002370814

## Description

### Field of the invention

The present invention provides methods for the prevention and treatment for renal disease. In particular, the invention provides methods of prevention and treatment of mammals, including humans, which are at risk of developing renal failure. This is generally in the field of treatment or prevention of acute renal failure by administration of the anti-apoptotic molecule survivin. The invention also provides the treatment of kidney transplants (renal allografts) to prolong survival of the graft during cold ischemia and immediately after transplantation.

### Background of the invention

Acute renal failure (ARF) leading to renal insufficiency is a common disorder, estimated to occur in at least 5% of all hospitalized patients, and in 30-50% of those admitted to the intensive care unit. Morbidity and mortality from ARF remain unacceptably high, and indeed, in spite of advances in supportive care, outcomes have not improved in the past 4 decades (27). The commonest cause of ARF - acute tubular necrosis (ATN) - is most frequently observed in the setting of sepsis, post-renal transplant, post-myocardial infarct, in the elderly with diminished fluid intake, and as a consequence of exposure to a wide range of toxins, including cis-platinum, aminoglycosides, amphotericin B, acyclovir, and radiocontrast agents (36, 42, 16). The notion that only severe renal failure impacts on long-term morbidity is dispelled by the fact that even modest degrees of renal insufficiency significantly increase the risk of death for critically ill patients (37). Despite intensive investigation into the pathophysiology of ARF, effective therapeutic strategies remain elusive. While the pathogenesis of renal tubular cell death in ATN is complex and varies depending on the etiology, severity, and stage of the illness, strong evidence supports the concept that apoptosis plays a central role (32, 31, 42, 33, 15). Ischemia-reperfusion induced ARF is associated with activation of caspases and prominent increases in renal tubular expression of several proapoptotic genes and/or proteins, including FADD, p53, Bad, Fas, and Smac/Diablo (15, 28, 21) (reviewed in (6)). At doses used clinically, cis-platinum induces ARF that is associated with caspase activation and histological changes consistent with renal tubular cell apoptosis (22). Post-renal transplant, apoptosis of donor kidney tubular epithelial cells and low Bcl-xL and Bcl-2 expression are associated with a high incidence of ARF (29, 35). Overall, the longheld view that cellular necrosis is the sole mechanism responsible for tubular epithelial cell death in ARF has thus been supplanted by a paradigm in which apoptosis plays a key role. Interfering with one or more pro-apoptotic pathways at crucial times during progression of ARF is therefore likely to be protective but it is not obvious which pathway or which molecule should be targeted to establish a treatment for ARF. Major gains have been made in elucidating the molecular mechanisms regulating apoptosis, and consequently, several molecular steps may be targeted to interfere with downstream activation of caspases. Survivin is a unique member of the inhibitor of apoptosis protein (IAP) family (reviewed in (25) and disclosed in WO9822589 and US6245523. It is minimally expressed in adult tissues, but abundant in most proliferating cells (2). Overexpression of survivin can protect cells from Fas- and injury-induced apoptosis (38), in part by interfering with effector caspases and likely stabilizing mitochondrial function, whereas suppression of survivin expression by anti-sense, ribozymes, or transgenic inactivation in mice leads to spontaneous apoptosis, and increased sensitivity to Fas and ischemia/hypoxia (7, 30, 19, 43, 9, 41). In contrast to other IAPs, survivin also plays a role in facilitating cell cycle progression, and furthermore is a chromosome passenger protein that is critical for regulation of mitosis and cytokinesis (41, 5). In the present invention we have utilized well-established murine models of acute renal failure (24) and transgenic mice that have diminished levels of survivin (9) to elucidate the role of survivin in the pathophysiology of ARF. We have surprisingly found that survivin, when delivered as a single dose prior to or at the onset of induced ARF effectively reduces renal tubular cellular damage and preserves renal function. It is known in the art that apoptosis plays a unique role in the pathogenesis of renal tubular cell death and it is also known that survivin, amongst many other inhibitory apoptosis protein, can protect cells against injury-induced apoptosis but it could however not be predicted that the overexpression of survivin can give a protection against the induction of acute renal failure. WO 98/22589 and Dohi T et al, 2004, Journal of Biological chemistry, 279(33) pp 34087-34090 disclose that survivin inhibits apoptosis

### Figure Legends

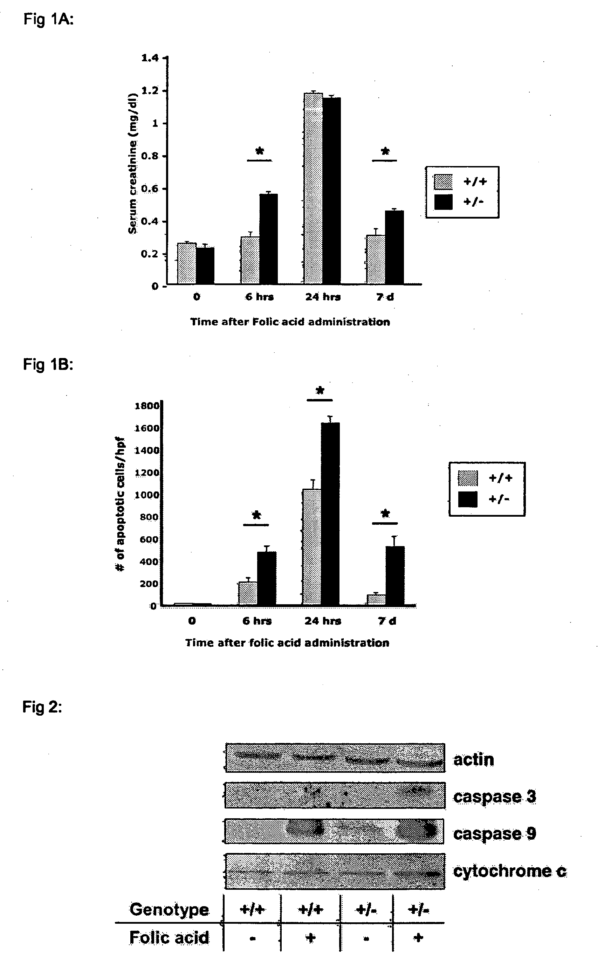

Figure 1: Survivin-dependent response to folic acid induced. Acute renal failure was induced with folic acid in *survivin*+/+ and *survivin+l-* mice. Serum creatinine (A) and the number of apoptotic cells detected by TUNEL staining of kidney sections (B) were quantified. *Survivin+l-* mice were more sensitive than *survivin*+*l*+ mice to induction of acute renal failure. Results reflect measures on a minimum of 3-5 mice. * p<0.05.
Figure 2: Activation of apoptosis after folic acid. Lysates of kidneys from *survivin+l+* and *survivin+l-* mice 24 hrs after administration of folic acid or saline were separated by SDS-PAGE and Western immunoblotted for detection of active fragments of caspase 3(17 Kd), caspase 9 (10 Kd), and release of cytochrome c. Detection of actin confirms equal loading. Activation of caspase 3 is only detected in lysates from *survivin*+/*-* mice exposed to folic acid, whereas caspase 9 is detectable in folic acid exposed to either genotype. Under baseline conditions, small amounts of caspase 9 are detected in lysates from *survivin*+*l*-mice. Cytochrome c release is more prominent in lysates from *survivin*+/*-*mice.

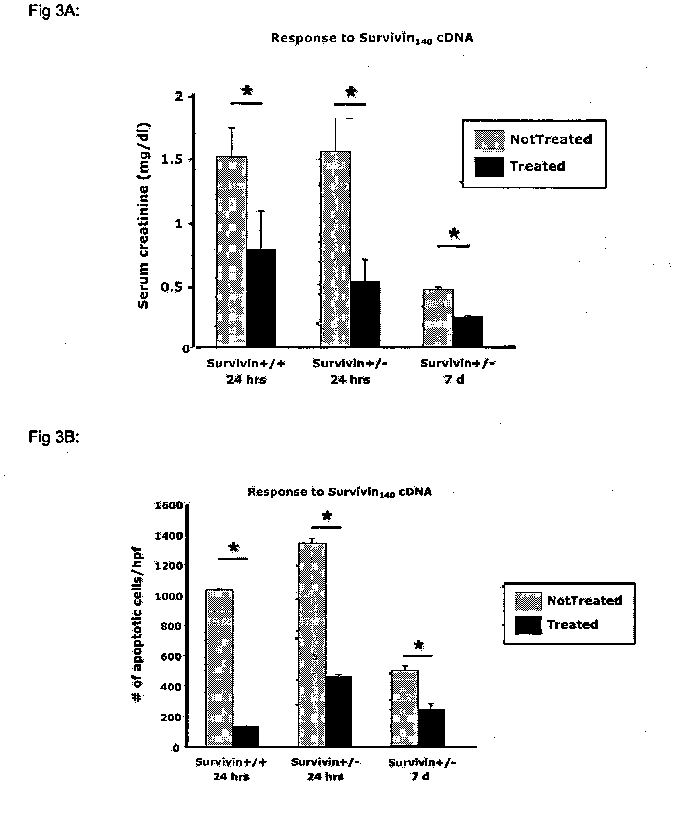

Figure 3: Protection from ARF after treatment with survivin_{140.} Via hydrodynamic gene delivery, mice were treated with either surviving₁₄₀ or with vector alone (control). 24 hrs later, folic acid was administered to induce ARF. A further 24 hrs or 7 days later, serum creatinine (B) and the number of apoptotic cells detected by TUNEL staining of kidney sections (B), were quantified. In both genotype mice, administration of survivin₁₄₀ results in significant improvements in both renal function as measure by serum creatinine, and in the number of apoptotic renal tubular cells.

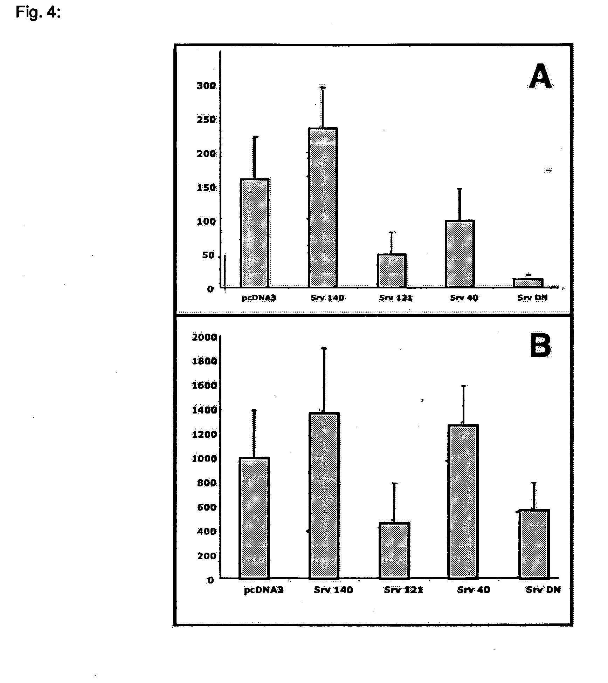

**Figure 4**: Effect of survivin administration on melanoma tumor growth. Survivin₁₄₀, survivin₁₂₁, survivin₄₀, survivin_{DN}, or a negative control (pcDNA3) was administered via hydrodynamic gene therapy as described in Methods. After 24 hrs, melanoma cells were injected subcutaneously into the left flank of each mouse. 6 days (A) and 13 days (B) later, tumor volumes were measured.

### Aims and detailed description of the invention

The mammalian renal system serves primary roles both in the removal of catabolic waste products from the bloodstream and in the maintenance of fluid and electrolyte balances in the body. Renal failures are, therefore, life-threatening conditions in which the build-up of catabolites and other toxins, and/or the development of significant imbalances in electrolytes or fluids, may lead to the failure of other major organ systems and death. As a general matter, renal failure is classified as "acute" or "chronic." As detailed below, the differences between these two conditions are herein further explained for the purpose of the invention. Acute renal failure is defined as an abrupt cessation or substantial reduction of renal function and, as many as 90-95% of cases may be secondary to trauma, surgery or another acute medical condition. Acute renal failure may be due to pre-renal causes (e.g., decreased cardiac output, hypovolemia, altered vascular resistance) or to post-renal causes (e.g., obstructions or constrictions of the ureters, bladder or urethra) which do not directly involve the kidneys and which, if treated quickly, will not entail significant loss of nephrons or other damage to the kidneys. Alternatively, acute renal failure may be due to intrinsic renal causes which involve a more direct insult or injury to the kidneys, and which may entail permanent damage to the nephrons or other kidney structures. Intrinsic causes of acute renal failure include but are not limited to infectious diseases (e.g., various bacterial, viral or parasitic infections), inflammatory diseases (e.g., glomerulonephritis, systemic lupus erythematosus), ischemia (e.g., renal artery occlusion), toxic syndromes (e.g., heavy metal poisoning, side-effects of antimicrobial treatments or chemotherapy), and direct traumas. The diagnosis and treatment of acute renal failure is as varied as its causes. In human patients, oliguria (urine output<400 ml/day) or anuria (urine output<50 ml/day) may be present in 50-70% of cases, BUN levels may climb 10-20 mg/dl/day or faster, plasma creatinine levels may climb 0.5-1.0 mg/dl/day, and metabolic acidosis is almost always present. If not treated, the electrolyte and fluid imbalances (e.g., hyperkalemia, acidosis, edema) associated with acute renal failure may lead to life-threatening arrhythmia, congestive heart failure, or multiple organ system failures. Chronic renal failure may be defined as a progressive, permanent and significant reduction of the glomerular filtration rate (GFR) due to a significant and continuing loss of nephrons. Chronic renal failure typically begins from a point at which a chronic renal insufficiency (i.e., a permanent decrease in renal function of at least 50-60%) has resulted from some insult to the renal tissues which has caused a significant loss of nephron units. The initial insult may or may not have been associated with an episode of acute renal failure. The progressive deterioration in renal function is slow, typically spanning many years or decades in human patients, but seemingly inevitable. The early stage of chronic renal failure typically begins when GFR has been reduced to approximately one-third of normal (e.g., 30-40 ml/min for an average human adult). As chronic renal failure progresses, and GFR continues to decline to less than 10% of normal (e.g., 5-10 ml/min), the subject enters end-stage renal disease (ESRD). During this phase, the inability of the remaining nephrons to adequately remove waste products from the blood, while retaining useful products and maintaining fluid and electrolyte balance, leads to a rapid decline in which many organ systems, and particularly the cardiovascular system, may begin to fail. For example, BUN and creatinine levels may be expected to rise and, at BUN levels of 60-100 mg/dl and serum creatinine levels of 8-12 mg/dl, a uremic syndrome will typically develop in which the kidneys can no longer remove the end products of nitrogen metabolism. At this point, renal failure will rapidly progress to death unless the subject receives renal replacement therapy (i.e., chronic hemodialysis, continuous peritoneal dialysis, or kidney transplantation). Approximately 600 patients per million receive chronic dialysis each year in the United States, at an average cost approaching $60,000-$80,000 per patient per year. Of the new cases of end-stage renal disease each year, approximately 28-33% are due to diabetic nephropathy (or diabetic glomerulopathy or diabetic renal hypertrophy), 24-29% are due to hypertensive nephrosclerosis (or hypertensive glomerulosclerosis), and 15-22% are due to glomerulonephritis. The 5-year survival rate for all chronic dialysis patients is approximately 40%, but for patients over 65, the rate drops to approximately 20%. In the last 5-10 years, several therapies have been evaluated to treat and/or prevent acute renal failure (ARF), none of which have been shown to be effective in humans. These include, for example, insulin growth factor I (11), lysophosphatidic acid (13), minocycline (23), interleukin-10 (14), antioxidants (39), parathyroid hormone-related protein, hepatocyte growth factor (HGF) (17), and atorvastatin (34). Recovery from acute renal failure may last from weeks to months, in proportion to a patient's age. In the meantime, acute dialysis is required on at least a tri-weekly basis. It would therefore represent a considerable saving, both in health care dollars as well as lives, if acute renal failure could be quickly aborted pharmacologically. Thus, there is clearly an urgent need for new agents that may be used singly or in combination, and these must be safe and efficacious.

We have found that in response to folic acid induced ARF, *survivin+*/*-* mice exhibited a worse functional and histological outcome compared to wild type sibling controls. Onset of ARF was more rapid in the *survivin*+/*-* mice. Although serum creatinine levels were similarly elevated in *survivin*+*l*+ and *survivin*+/*-* mice at 24 hrs, the morphology of the kidneys of the *survivin*+*l-*mice was notably worse, with more evidence of renal tubular epithelial cell necrosis and apoptosis. By 7 days after injection of folic acid, renal function and morphology of the *survivin*+*l*+ mice returned almost to normal, whereas the recovery time of the *survivin*+*l*- mice was delayed. The contribution of apoptosis to the more rapid and severe progression of renal failure in the *survivin*+/- mice was substantiated by TUNEL staining kidney sections, and was also documented biochemically by greater increases in caspase-3 and caspase-9 activation, and release of cytochrome c.

In view of the profound effect that low levels of survivin had on renal function when exposed to folic acid, we tested the administration of survivin in established mouse models for acute renal failure. As fully described in the examples the present invention shows that survivin is an effective therapeutic target to treat and/or prevent renal failure. More particularly, the invention shows the importance of the inhibitor of apoptosis protein (IAP), survivin, in protecting the kidney against the induction of acute renal failure, and furthermore also demonstrates that therapy with functional forms of survivin, in which the BIR domain is intact, also has therapeutic utility.

A first aspect of the invention is the use of survivin, or a functional fragment thereof, or a variant thereof having the biological activity of survivin or a nucleotide sequence encoding survivin said fragment or said variant for the manufacture of a medicament prevent and/or to treat renal failure. In a particular embodiment survivin comprises the amino acid sequence depicted in SEQ ID NO: 1 is used for the manufacture of a medicament to prevent and/or to treat renal failure. SEQ ID NO: 1 represents the human amino acid sequence (128 amino acids) of survivin that is equivalent to the murine counterpart survivin₁₂₁ and has lost its C-terminal coiled-coil structure. The therapeutic efficacy of for example human survivin₁₂₈, which lacks the C-terminus coiled-coil structure that links the function of survivin to the cell cycle (4), is particularly relevant in the design of safe human therapies. Survivin is highly expressed in essentially all tumors (reviewed in (25)), and concerns that treatment with survivin might induce tumor growth, are soundly based. Indeed, in vitro studies show that survivin promotes cell proliferation in hepatocellular carcinoma (20), while *in vivo,* survivin may oppose the elimination of cancerous cells by p53 (18). This concern might be mitigated if one could identify those domains of survivin that do not induce tumor formation. For this reason, we evaluated the in vivo proliferative response of a melanoma cell line to different forms of survivin delivered via gene therapy. As expected, in mice survivin₁₄₀ resulted in larger tumors, survivin₄₀ had no effect, and the dominant negative survivin_{DN} suppressed tumor growth. Notably, in mice survivin₁₂₁ also resulted in smaller tumors after 6 and 13 days. The mechanism by which survivin₁₂₁ inhibited tumor growth has not yet been elucidated, but we hypothesize that it may be related to its dimerization with survivin₁₄₀. In any case, the findings show that the survivin₁₂₁ equivalent (for example in human this is survivin₁₂₈) or fragments of survivin that retain the BIR domain, yet are lacking the coiled-coil domain, are safe and effective for clinical use as an inhibitor of apoptosis in ARF. In another embodiment the full length splice form of survivin (survivin₁₄₂ Genbank accession number: AAC51660 and described in Ambrosini G. et al (1997) Nat. Med. 3(8) 917-921 is used for the manufacture of a medicament to prevent and/or to treat renal failure. In yet another particular embodiment the functional fragment of survivin is a fragment that comprises the amino acid sequence as depicted in SEQ ID NO: 3 and that is able to inhibit apoptosis. A functional fragment of survivin is a fragment comprising the BIR domain of survivin and is able to inhibit apoptosis. The amino acid sequence of the BIR domain is depicted in SEQ ID NO: 3, the nucleotide sequence of said BIR domain is depicted in SEQ ID NO: 4. Still other survivin homologues which can be used, particularly in veterinary medicine, for the manufacture of a medicine to treat renal failure, more particularly acute renal failure are for example survivin from *Felis catus* (cat) Genbank accession number AB182320.1 and survivin from *Canis familiaris* (dog) Genbank accession numbers AY741504.1, NM_001003348.1, AB180206.1, AB095108.1, AB095108 and NM_001003019. Still other functional fragments of survivin and homologues thereof which can be used in the present invention are functional splice variants of survivin described in Conway EM et al (2000) Blood 95, 1435-42; Mahotka C et al (1999) Cancer Res. 59, 6097-102 and Caldas H et al (2005) Mol. Cancer 4, 11. Apoptosis can be measured by various assays described in the art. One disclosed method is the influence of recombinant survivin or fragments thereof on the inhibition of apoptosis that is induced by growth factor (IL-3) withdrawal in pre-B cell transfectants (Ambrosini G. et al (1997) Nat. Med. 3(8), 917-921. Survivin is disclosed in WO9822589 and US6245523. Survivin, or a functional fragment thereof, or a variant thereof may be fused or chemically coupled to a sequence facilitating transduction of the fusion or chemical coupled proteins into eukaryotic cells. Sequences, facilitating protein transduction are known to the person skilled in the art and include, but are not limited to Protein Transduction Domains. Preferably, said sequence is selected from the group comprising of the HIV TAT protein, a polyarginine sequence, penetratin and pep-1. Still other commonly used cell-permeable peptides (both natural and artificial peptides) are disclosed in Joliot A. and Prochiantz A. (2004) Nature Cell Biol. 6 (3) 189-193. A second aspect of the invention is the use of a nucleotide sequence encoding survivin comprising SEQ ID NO: 2, or a functional fragment comprising SEQ ID NO: 4 or a variant thereof, for the manufacture of a medicament to prevent and/or to treat kidney failure. Variants are polypeptides with at least 65% identity on amino acid level, preferably 70% identity, as measured by BLAST (Altschul SF et al., (1997) Nucleic Acids Res 25, 3389-3402). Variants have one or more common characteristics, such as biological activity, immunological reactivity, conformation etc. A functional fragment or variant of the survivin protein or of the nucleotide sequence encoding survivin, as used here, is a protein sequence, having some of the common characteristics of the survivin protein or a nucleic acid sequence that encodes a functional fragment or variant of survivin, as described above.

Said nucleic acid sequence may be cloned in a suitable expression vector, as will be detailed below.

In case a nucleic acid is used, said medicament is preferably intended for delivery of said nucleic acid into the cell, in a gene therapy treatment. A large number of delivery methods are well known to those of skill in the art. Preferably, the nucleic acids are administered for *in vivo* or *ex vivo* gene therapy uses. Non-viral vector delivery systems include DNA plasmid, naked nucleic acid, and nucleic acid complexed with a delivery vehicle such as a liposome. Viral vector delivery systems include DNA and RNA viruses, which have either episomal or integrated genomes after delivery to the cell. Methods of non-viral delivery of nucleic acids include lipofection, microinjection, biolistics, virosomes, liposomes, immunoliposomes, polycation or lipid: nucleic acid conjugates, naked DNA, artificial virions, and agent-enhanced uptake of DNA. Lipofection is described in, e.g., US Pat. No. 5,049,386, US Pat No. 4,946,787; and US Pat. No. 4,897,355 and lipofection reagents are sold commercially (e.g., Transfectam^{™} and Lipofectin™). Cationic and neutral lipids that are suitable for efficient receptor-recognition lipofection of polynucleotides include those of Flegner, WO 91/17424, WO 91/16024. Delivery can be to cells (*ex vivo* administration) or target tissues (*in vivo* administration). The preparation of lipid: nucleic acid complexes, including targeted liposomes such as immunolipid complexes, is well known to one of skill in the art (*see, e.g*., Crystal, 1995; Blaese *et al.,* 1995; Behr, 1994; Remy *et al.,* 1994; Gao and Huang, 1995; U.S. Pat Nos. 4,186,183, 4,217,344, 4,235,871, 4,261,975, 4,485,054, 4,501,728, 4,774,085, 4,837,028, and 4,946,787). The use of RNA or DNA viral based systems for the delivery of nucleic acids take advantage of highly evolved processes for targeting a virus to specific cells in the body and trafficking the viral payload to the nucleus. Viral vectors can be administered directly to patients (*in vivo*) or they can be used to treat cells *in vitro* and the modified cells are administered to patients (*ex vivo*)*.* Conventional viral based systems for the delivery of nucleic acids include amongst others retroviral, lentivirus, adenoviral, adeno-associated and herpes simplex virus vectors for gene transfer. Viral vectors are currently the most efficient and versatile method of gene transfer in target cells and tissues. Integration in the host genome is possible with the retrovirus, lentivirus, and adeno-associated virus gene transfer methods, often resulting in long-term expression of the inserted transgene. Additionally, high transduction efficiencies have been observed in many different cell types and target tissues.

In cases where transient expression of the nucleic acid is preferred, adenoviral based systems, including replication deficient adenoviral vectors may be used. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors, including recombinant adeno-associated virus vectors are also used to transduce cells with target nucleic acids, e.g., in the *in vitro* production of nucleic acids and peptides, and for *in vivo* and *ex vivo* gene therapy procedures (see, *e.g.,* U.S. Patent No. 4,797,368; WO 93/24641; Kotin, 1994; The construction of recombinant AAV vectors is described in a number of publications, including U.S. Pat. No. 5,173,414; Hermonat & Muzyczka, 1984; Samulski *et al*., 1989).

Gene therapy vectors can be delivered *in vivo* by administration to an individual patient, typically by systemic administration (e.g., intravenous, intraperitoneal, intramuscular, intratracheal, subdermal, or intracranial infusion) or topical application.

In a particular embodiment the invention also envisages the use of a hydrodynamic gene therapeutic method. Hydrodynamic gene therapy is disclosed in US6627616 (Mirus Corporation, Madison) and involves the intravascular delivery of non-viral nucleic acids encoding survivin or a functional fragment or a variant thereof whereby the permeability of vessels is increased through for example the application of an increased pressure inside said vessel or through the co-administration of vessel permeability increasing compounds such as for example papaverine.

Alternatively, vectors can be delivered to cells *ex vivo,* such as cells explanted from an individual patient (e.g., lymphocytes, bone marrow aspirates, tissue biopsy) or universal donor hematopoietic stem cells, followed by reimplantation of the cells into a patient, usually after selection for cells which have incorporated the vector. *Ex vivo* cell transfection for diagnostics, research, or for gene therapy (e.g., via re-infusion of the transfected cells into the host organism) is well known to those of skill in the art. In a preferred embodiment, cells are isolated from the subject organism, transfected with a nucleic acid (gene or cDNA), and re-infused back into the subject organism (e.g., patient). Various cell types suitable for *ex vivo* transfection are well known to those of skill in the art (*see, e.g.,* Freshney *et al.,* 1994 and the references cited therein for a discussion of how to isolate and culture cells from patients).

In a further embodiment the invention provides a method for the production or manufacture of a medicament or a pharmaceutical composition comprising survivin or a functional fragment or variant thereof and further more mixing said polypeptide with a pharmaceutically acceptable carrier. Alternatively, the pharmaceutical composition may comprise an survivin inducing compound in stead of survivin itself. In a preferred embodiment a polypeptide comprising survivin or a functional fragment or a variant thereof is a recombinant protein. The recombinant protein may be manufactured using recombinant expression systems comprising bacterial cells, yeast cells, animal cells, insect cells, plant cells or transgenic animals or plants. The recombinant protein may be purified by any conventional protein purification procedure close to homogeneity and/or be mixed with additives.

The administration of a pharmaceutical composition comprising survivin or a functional fragment or variant thereof may be by way of oral, inhaled or parenteral administration. The active compound may be administered alone or preferably formulated as a pharmaceutical composition. A unit dose will normally contain 0.01 to 50 mg for example 0.01 to 10 mg, or 0.05 to 2 mg of compound or a pharmaceutically acceptable salt thereof. Unit doses will normally be administered once or more than once a day, for example 2, 3, or 4 times a day, more usually 1 to 3 times a day, such that the total daily dose is normally in the range of 0.0001 to 1 mg/kg; thus a suitable total daily dose for a 70 kg adult is 0.01 to 50 mg, for example 0.01 to 10 mg or more usually 0.05 to 10 mg. It is greatly preferred that the compound or a pharmaceutically acceptable salt thereof is administered in the form of a unit-dose composition, such as a unit dose oral, parenteral, or inhaled composition. Such compositions are prepared by admixture and are suitably adapted for oral, inhaled or parenteral administration, and as such may be in the form of tablets, capsules, oral liquid preparations, powders, granules, lozenges, reconstitutable powders, injectable and infusable solutions or suspensions or suppositories or aerosols. Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well-known methods in the art. Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate. These solid oral compositions may be prepared by conventional methods of blending, filling, tabletting or the like. Repeated blending operations may be used to distribute the active agent throughout those compositions employing large quantities of fillers. Such operations are, of course, conventional in the art. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents. Oral formulations also include conventional sustained release formulations, such as tablets or granules having an enteric coating. Preferably, compositions for inhalation are presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns, for example between 1 and 5 microns, such as between 2 and 5 microns. Alternatively, coated nanoparticles can be used, with a particle size between 30 and 500 nm. A favored inhaled dose will be in the range of 0.05 to 2 mg, for example 0.05 to 0.5 mg, 0.1 to 1 mg or 0.5 to 2 mg. For parenteral administration, fluid unit dose forms are prepared containing a compound of the present invention and a sterile vehicle. The active compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum. Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound. Where appropriate, small amounts of bronchodilators for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included. As is common practice, the compositions will usually be accompanied by written or printed directions for use in the medical treatment concerned.

With regard to the protein transduction with survivin or functional fragments or variants thereof into target cells, it has been shown that a series of small protein domains, termed protein transduction domains (PTDs), cross biological membranes efficiently and independently of transporters or specific receptors, and promote the delivery of peptides and proteins into cells. For example, the TAT protein from human immunodeficiency virus (HIV-1) is able to deliver biologically active proteins in vivo. Similarly, the third alpha-helix of Antennapedia homeodomain, and VP22 protein from herpes simplex virus promote the delivery of covalently linked peptides or proteins into cells {reviewed in Ford KG et al (2001) Gene Ther. 8, 1-4). Protein delivery based on a short amphipathic peptide carrier, Pep-1, is efficient for delivery of a variety of peptides and proteins into several cell lines in a fully biologically active form, without the need for prior chemical covalent coupling (Morris MC et al, (2001) Nat. Biotechnol. 19, 1173-1176). The capacity of VP22 chimeric proteins to spread from the primary transduced cell to surrounding cells can improve gene therapy approaches (Zender L et al (2002) Cancer Gene Ther. 9, 489-496). Thus in a preferred embodiment a synthetic or recombinant survivin or functional fragment or variant fused to a protein transduction domain is used for the manufacture of a medicament to treat and/or to prevent renal failure. In another preferred embodiment the BIR domain of survivin coupled to a protein transduction domain is used for the manufacture of a medicament to treat and/or to prevent renal failure.

The present invention is further illustrated by way of examples, which are not considered to be limiting.

### Examples

### 1.Survivin+l- mice are more susceptible to folic acid induced ARF

Folic acid was administered to induce ARF with tubular epithelial cell death. *Survivin*+*l*+ and *survivin*+/- mice were evaluated at 6 hrs, 24 hrs and 7 days (Figure 1). Under baseline conditions, there were no discernible differences between the *survivin*+*l*+ and the *survivin*+*l-*mice in terms of renal function (serum creatinine), or histologic appearance of the kidneys, as assessed by H&E staining and evidence of apoptosis (Figure 1). At 6 hrs, the serum creatinine level of *survivin*+*l*+ mice did not change from baseline. By 24 hrs, the serum creatinine level became notably elevated, but by 7 days, renal function as measured by serum creatinine, had returned to normal in the *survivin*+*l*+ mice, a pattern of recovery that is typical for folic acid induced ARF in mice (12).

The response to folic acid in the *survivin*+*l*- mice was notably different (Figure 1). A more rapid onset of ARF was observed, as the serum creatinine became significantly elevated at 6 hrs. By 24 hrs, renal function had deteriorated to a similar extent to that observed with the *survivin*+*l*+ mice. But at 7 days, rather than a full recovery in renal function, the serum creatinine remained significantly elevated in the *survivin*+*l*- mice. Overall, the results indicate that diminished levels of survivin in the *survivin*+/- mice confer increased sensitivity to folic acid induced ARF.

### 2. Low levels of survivin render renal tubular cells sensitive to apoptosis induced by folic acid

The mechanisms by which low levels of survivin result in increased sensitivity to folic acid induced ARF were evaluated. In keeping with the absence of early changes in serum creatinine in the *survivin*+*l*+ mice, the kidneys exhibited no histologic evidence by H&E staining, of damage 6 hrs after folic acid injection. Nonetheless, TUNEL staining revealed a 20-fold increase in the number of apoptotic renal tubular epithelial cells as compared with baseline (Figure 1 B), highlighting the early onset of apoptosis following this toxic injury. By 24 hrs, when the serum creatinine was elevated, the number of apoptotic cells increased further. Otherwise, the most prominent histologic finding in the kidneys was that the collecting ducts appeared dilated. A minority of the renal tubular epithelial cells exhibited loss of brush borders and condensed nuclei. By 7 days, when renal function had returned to normal, the kidneys appeared histologically normal by H&E staining (not shown), although the number of apoptotic cells was still somewhat elevated, but approaching normal (Figure 1 B).

Histologic changes were much more dramatic in the *survivin*+/- mice throughout the study period. 6 hrs post folic acid, H&E staining revealed more evidence of renal tubular cell damage, with condensed nuclei, and loss of brush borders. There was a 47-fold increase in the number of apoptotic cells as compared to baseline, and this was significantly more than that which was observed with the *survivin*+/+ kidneys at the same time point (p<0.05) (Figure 1B). By 24 hrs, the kidneys of *survivin*+/- mice exhibited diffuse and major histologic changes reflecting extensive tubular epithelial cell damage, with evidence of both necrosis and apoptosis, as is commonly seen with this model (12). The majority of renal tubular cells were swollen, with accumulation of apical cytoplasmic vacuoles, and loss of brush borders. Tubular epithelial cell nuclei were condensed, with evidence of apoptotic bodies, accompanied by shedding of cells and cellular debris and/or casts into the collecting ducts. TUNEL staining of kidney sections from *survivin*+/- mice confirmed the significant increase in renal tubular cell death relative to that observed in the kidneys of *survivin*+*l*+ mice at 24 hrs. These findings persisted until at least 7 days after folic acid was administered (Figure 1 B) at which time there was still a significant increase in apoptotic cells.

To further confirm activation of apoptotic pathways, we performed Western immunoblots of kidney lysates. Under baseline conditions, caspase-3 activation was not detectable in the kidneys of either genotype mice, and there was minimal detection of caspase-9 activation in the kidney lysates of the *survivin*+/*-* mice. 24 hrs after folic acid, prominent activation of caspase-9 in the kidney lysates of both *survivin*+*l*+ and *survivin*+/- mice was detected. Caspase-3 activation was only detectable in the lysates from the *survivin+*/*-* mice (Figure 2). The data demonstrate that survivin plays an important role in protecting renal tubular cells against apoptosis associated with ARF, and that heterozygous deficiency of survivin enhances the sensitivity of the kidneys to toxin induced, caspase-mediated cell death.

### 3. Gene therapy with survivin₁₄₀ or survivin₁₂₁ prevents folic acid induced acute tubular necrosis

Based on the preceding results, we predicted that over-expression of survivin might be protective against ARF. To over-express survivin in mice, we used hydrodynamic gene delivery, in which an expression plasmid vector is rapidly infused in a large volume intravenously into the tail vein. This method has been successfully utilized to deliver a variety of genes in mice, with persistence in elevated gene expression for over 5-7-days (12), and up to 4 months (1). In preliminary studies, we used hydrodynamic gene delivery of a vector with a CMV promoter driving lacZ to assess tissue distribution. At 6, 24 and 48 hrs, beta-galactosidase was detected in several tissues, with diffuse and prominent expression in the tubular epithelial cells of proximal and distal collecting ducts of the kidneys. A transient 1.2 to 1.4 fold increase in serum levels of the liver enzyme, alanine aminotransferase, was observed 24 hrs after gene delivery, but this normalized at 48 hrs. We have previously reported that, in the mouse, there are three distinct survivin mRNAs that encode functionally distinct proteins (8). Both survivin₁₄₀ (the full-length form) and survivin₁₂₁ retain the BIR domain that is crucial for interfering with caspase activation, whereas survivin₁₂₁ lacks the C-terminal coiled-coil domain which functionally links survivin to the cell cycle. Survivin₄₀ lacks both the coiled-coil domain and the BIR domain, and thus has no known independent anti-apoptotic function (8).

The gene delivery method was first used to treat *survivin*+*l*+ mice with survivin₁₄₀ or control 24 hrs prior to folic acid injection. A further 24 hrs and 7 days later, renal function and pathology were assessed (Figure 3). Expression of survivin in renal tubular cells in survivin-treated mice 24 hours after folic acid injection was confirmed by immunostaining kidney sections with specific anti-survivin antibodies. Several parameters indicated a beneficial response to the treatment with survivin₁₄₀ 24 hrs after folic acid. Mice which were pretreated with survivin₁₄₀ had significantly lower levels of serum creatinine (0.7 mg/dl treated *versus* 1.5 mg/dl control, p<0.05, n=3 in each group) (Figure 3A), less Bad positive renal tubular cells (132 ± 38 treated *versus* 250 + 25 control, p<0.05), and significantly fewer apoptotic renal tubular cells (129 + 11 treated *versus* 1030 + 10 control, p<0.05) (Figure 3B). Furthermore, survivin₁₄₀ pre-treatment prevented dilatation of the collecting ducts, renal tubular cell swelling, and brush border changes, and decreased the number of cells with condensed nuclei. The effect of over-expressing survivin₁₄₀ in *survivin*+*l*- mice was also evaluated. Similar to the response in *survivin*+/+ mice, pretreatment with survivin₁₄₀ protected the mice, as assessed 24 hrs after folic acid, from ARF, maintaining the serum creatinine levels in the normal range, significantly diminishing the number of Bad positive cells (203 + 62 control *versus* 133 + 15), and apoptotic renal tubular cells, and notably ameliorating the extent of renal tubular cell damage (Figure 3). Since the renal function of *survivin*+/- mice remained disturbed until at least 7 days post-folic acid injection (in contrast to *survivin*+*l*+ mice, which had recovered by then), we could also evaluate the response to pretreatment with surviving₁₄₀ over a longer interval. After 7 days, serum creatinine levels decreased from 0.45 to 0.24 mg/dl in treated *versus* control mice, respectively (p<0.05), and the number of apoptotic renal tubular cells were significantly diminished, showing that the protective effects of survivin administration are sustainable (Figure 3). When *survivin*+*l*- mice were treated with survivin₁₄₀ at the same time as folic acid was administered, serum creatinine levels were significantly improved as compared with treatment with vector alone (0.36 ± 0.2 mg/ml *versus* 1.81 ± 0.3 mg/ml, respectively).

However, when mice were treated with survivin₁₄₀ 2 hrs after administration of folic acid, serum creatinine levels remained elevated. The results indicate that prior to or at the onset of induction of ARF with folic acid, treatment with survivin₁₄₀ is beneficial. We also assessed the effects of other isoforms of survivin. Administration of survivin₁₂₁ cDNA to *survivin+l-* mice 24 hrs prior to folic acid injection also protected them against ARF as assessed 24 hrs after the induction procedure, preventing a rise in serum creatinine (0.65 ± 0.2 mg/dl treated *versus* 1.5 ± 0.2 mg/dl control, p<0.05, n=3 in each group), and significantly diminishing the number of apoptotic renal tubular cells. In fact, there was no discernible difference in response to therapy with survivin₁₄₀ and surviving₁₂₁. In contrast, pre-treatment of *survivin*+*l*+ mice with survivin₄₀ provided no protection against folic acid-induced ARF.

### 4. Effect of survivin on tumor growth

Survivin is highly expressed in most tumors (25), and *in vivo* administration of full-length forms may enhance tumor growth. To test the tumor-promoting capacity of different forms of survivin, we used an *in vivo* xenotransplant model. Melanoma cells were injected subcutaneously in nude mice 24 hrs after different forms of survivin were administered via hydrodynamic gene delivery (Figure 4). Compared with vector alone, survivin₁₄₀ caused an increase in tumor size, survivin₄₀ had no effect, and the dominant negative survivin_{DN} suppressed tumor growth. Interestingly, survivin₁₂₁ also suppressed tumor growth, although not to the same extent as the dominant negative form. The data show that survivin₁₂₁ does not induce tumor growth, but does protect against ARF, and thus is a safe alternative to survivin₁₄₀.

### 5. Gene delivery of survivin₁₄₀ protects against acute renal failure in a murine model of renal ischemia-reperfusion injury

Survivin₁₄₀ was administered via hydrodynamic gene therapy (treatment) or with the control vector alone (sham) in mice. After 24 hrs, acute renal ischemia-reperfusion injury was surgically induced in wild-type mice by established techniques. Briefly, mice were anesthetized and placed on a temperature-regulated dissecting table to maintain rectal temperature at 37°C (according to Deng J et al (2001) Kidney Int. 60:2118-2128 and Singbartl K et al (2000) FASEB J 1448-54). The renal pedicles were clamped bilaterally for 32 mins, and then released, allowing reperfusion for an additional 48 hrs, after which bloods were drawn to measure serum levels of creatinine, and the kidneys were surgically removed to assess them histologically. This murine model of bilateral renal ischemia/reperfusion has the advantage of more closely resembling human disease with acute tubular necrosis and apoptosis of renal tubular epithelial cells, associated with influx of leukocytes, deposition of complement, and elevation of serum cytokines. The results clearly indicate that administration of survivin₁₄₀ via hydrodynamic gene delivery protects against acute renal failure. As seen in Table 1, serum creatinine levels remain close to normal in the treatment group(n=5 in each group), whereas sham-treatment with an empty vector provides no protection against a rise in serum creatinine. Histologic findings also clearly show that survivin₁₄₀ protects the kidney from ischemia-reperfusion induced damage to the renal tubules.

**Table 1: Serum creatinine levels 48 hrs after induction of bilateral ischemia-reperfusion injury (32' ischemia) by transient bilateral clamping of renal pedicles. Pre-treatment with survivin₁₄₀, administered by hydrodynamic gene delivery 24 hrs prior to ischemia, prevents rise in serum creatinine.**

| | **Treatment** | **Sham** |
|---|---|---|
| **Serum creatinine** | 0.37 ± 0.1 (SEM) | 1.21 ± 0.2 (SEM) |

### 6. Chemotherapy-induced acute renal failure

Cis-platinum is an antineoplastic agent, commonly used to treat solid tumors. Administration in humans is dose-restricted due to nephrotoxicity, inducing RTC apoptosis by activation of caspase-3. To induce acute renal failure in mice, we are injecting them with 20 mg/kg of cisplatinum. At 72 hrs, histologic changes include RTC apoptosis in the proximal and straight tubules, tubular casts, and peritubular leukocyte accumulation. We are therefore evaluating the functional and histologic response of rescuing and/or preventing the onset of acute renal failure by administration of survivin isoforms described herein.

### 7. Acute renal failure induced by kidney transplantation in rodents

Apoptosis plays an important role in the outcome of kidney transplantation. Syngeneic rats are being used as donors and recipients. Donor kidneys are harvested after damping of the infradiaphragmatic aorta. The kidneys are cold perfused (Fuller TF et al (2003) Transplantation. 2003;76:1594-1599 and Fuller TF et al (2004) J Urol. 2004;171:1296-1300) and stored for varying periods at 4°C. In the recipient animal, both kidneys are removed and the donor kidney is transplanted (Fuller TF et al (2003) Transplantation. 2003;76:1594-1599 and Fuller TF et al (2004) J Urol. 2004;171:1296-1300). Survivin and fragments and isoforms described herein, or a dominant negative form are introduced into the donor kidneys or rat recipient via gene delivery before or after transplantation to test effects on transplant or storage-induced apoptosis. After transplantation, biopsy specimens and blood samples are obtained to monitor renal structure/function. After 1-4 weeks, animals are sacrificed for more extensive studies to evaluate for renal damage and activation of relevant biochemical pathways. Kidney transplantation in mice is carried out according to the microsurgical techniques described in Zhang Z et al (1995) Microsurgery 16(2):103-9.

### 8. Tranduction of bone marrow stem cells with survivin

Recent evidence supports the concept that in response to injury, the kidney has a remarkable capacity for repair, and hematopoietic stem cells from the bone marrow may contribute to regeneration of RTCs (Lin F et al (2003) J Am Soc Nephrol. 14:1188-1199, Poulsom R et al (2003) J Am Soc Nephrol. 2003;14 Suppl 1:S48-54). Notably, survivin is expressed in pluripotent stem cells from the bone marrow (c-kit+, Lin-) (Fukuda S et al (2004) Blood 3:120-127) where it is essential for normal cellular proliferation and differentiation. According to the present findings it is reasonable to believe that stem cells that express high levels of survivin when mobilized to the kidney post-injury can transdifferentiate into RTCs and improve recovery from ATN. We are presently testing whether upregulation of survivin in hematopoietic stem cells enhances recovery from ARF. Therefore, bone marrow progenitor stem cells are isolated (Jiang Y et al (2002) Exp Hematol. 30:896-904 and Van Damme A et al (2002) Curr Gene Ther. 2:195-209) and transfected by retroviral techniques with a bicistronic plasmid containing an IRES-EGFP preceded by the survivin cDNA encoding either fragments or variants or dominant negative forms of survivin as herein described. c-kit+, Lin-cells are isolated and FACS sorted (Fukuda S et al (2004) Blood103:120-127 and Lin F et al (2003) J Am Soc Nephrol. 14:1188-1199) from the BM, and assessed for survivin expression by RT-PCR, and immunostaining prior to use. The transfected progenitor BM stem cells (Lin F et al (2003) J Am Soc Nephrol. 14:1188-1199) are transplanted into syngeneic mice prior to or 24 hrs after induction of ARF with toxin, ischemia-reperfusion, or other models described herein. Observation periods extend for up to 4 weeks. In addition to the functional, structural and molecular studies described above, kidney sections will be evaluated for integration of GFP-positive cells, and these are further characterized immunologically for stem cell and RTC markers.

### Materials and methods

### 1.Transgenic Mice

Generation of *survivin*+/- mice by homologous recombination in embryonic stem cells has been reported (9). Transgenic mice were maintained on a Swiss:129s (50:50) genetic background, and housed in a specific pathogen-free environment. The *survivin*+/- mice express approximately 50% levels of survivin mRNA, and under non-stress conditions, have no phenotypic abnormalities (9, 10). Experiments were performed with 10-12 week old, 25-30 gm male mice. *Survivin*+*l*+ littermates were used as controls for experiments on *survivin*+*l-*mice. Studies were approved by the animal ethics committee at the University of Leuven.

### 2. Models for the induction of Acute Renal Failure

ARF was induced by a single intraperitoneal (ip) injection of folic acid 250 mg/kg body weight, dissolved in 150 µl sodium bicarbonate (NaHCO₃). Control animals were administered 150 µl sodium bicarbonate ip (24).

### 3. Plasmid Preparation and Hydrodynamic Gene Delivery

The cDNAs encoding full-length murine survivin (survivin₁₄₀), survivin₁₂₁ and survivin₄₀ (8), were each cloned into the expression vector pcDNA3 (Invitrogen, San Diego, CA), resulting in the vectors survivin₁₄₀/pcDNA3, survivin₁₂₁/pcDNA3, and survivin₄₀/pcDNA3. The cDNA encoding a dominant negative survivin, survivin_{DN}, in which the threonine at amino acid position 34 is substituted for an alaninie, was also subcloned into pcDNA3. For *in vivo* gene delivery, plasmid DNA 25 µg in 2 ml of saline, was injected in 5-6 seconds via the tail vein of mice (1, 12). Vector alone was injected for non-treatment controls.

### 4. Renal Function and Preparation of Kidneys for Histo-Pathologic Analyses

At different times after induction of ARF, mice were anesthetized. The chest wall and abdomen were surgically exposed, and blood was drawn from the inferior vena cava for measurement of serum creatinine. Mice were perfused transcardially with saline, after which the left kidney was removed, frozen in liquid nitrogen and stored at -80°C. Perfusion was resumed with zinc-buffered formalin (Z-fix, Anatech Ltd., Battlecreek, MI), after which the right kidney was removed for histology.

Kidneys were incubated overnight in Z-fix, dehydrated through increasing ethanol concentrations, embedded in paraffin wax, and prepared for histologic sectioning. 7-µm sections were stained for haematoxylin and eosin (H&E), or incubated after antigen retrieval with specific antibodies, followed by addition of appropriate horse-radish peroxidase (HRP) conjugated secondary antibodies, and visualization by immunoperoxidase staining. Control primary antibodies were used to exclude non-specific staining.

### 5. Detection of Apoptosis

Apoptosis was detected *in situ* by staining deparaffinized sections using the ApopTag Peroxidase In Situ Apoptosis Detection Kit (Chemicon, Hofheim, Germany) according to the manufacturer's instructions. Quantification of apoptosis by an investigator blinded to experimental conditions and mouse genotype was accomplished by microscopically determining the number of peroxidase positive cells in 2 non-adjacent sections per mouse in 5 high-power fields (hpf) per section. Results are expressed as the mean ± standard error of the mean (SEM).

### 6. SDS-PAGE and Western Immunoblots

Kidneys were lysed and homogenized on ice in a solution containing 1% Triton X-100, 150 mM NaCl, 0.1 mM ethylenediaminetetraacetate, 20 mM Hepes pH 7.5, 20% glycerol and 1 mM MgCl₂ in the presence of protease inhibitors. Protein content of cleared lysates were quantified with the BCA kit (Promega, Leiden, the Netherlands). 100 µg of each were separated by SDS-PAGE under reducing conditions and transferred to a nitrocellulose membrane which was blocked with 5% non-fat dried milk powder in PBS with 0.1% Tween 20 and incubated for 2 to 24 hours with the primary antibody. After washing and incubation of the membrane with the appropriate HRP-conjugated secondary antibody, detection was accomplished using the enhanced chemiluminescence method (Amersham-Biosciences, Freiburg, Germany). Equal loading was confirmed by re-blotting the membranes for detection of actin.

### 7. Melanoma tumor growth

B6 melanoma cells were cultured in DMEM with 10% FBS until preconfluent, trypsinized, washed and suspended in PBS. 24 hrs after hydrodynamic gene delivery of survivin forms, 6-8 week old, female nude mice were injected subcutaneously in the left flank with 2 million melanoma cells in a volume of 200 µl PBS. Tumor volumes were quantified after 6 and 13 days by an investigator blinded to which cDNA was delivered. At day 13, mice were sacrificed and tumors were excised and weighed.

### 8. Statistical Analyses

Statistics were performed with InStat® software (MacKiev Company, Cupertino,CA). Data was tested using one-way ANOVA, followed by Tukey-Kramer multiple comparisons test. P-values < 0.05 were considered significant.

### References

Alino, SF, Crespo, A & Dasi, F: Long-term therapeutic levels of human alpha-1 antitrypsin in plasma after hydrodynamic injection of nonviral DNA. Gene Ther, 10:1672-9, 2003.
Altieri, DC: Survivin, versatile modulation of cell division and apoptosis in cancer. Oncogene, 22:8581-9, 2003.
Altznauer, F, Martinelli, S, Yousefi, S, Thurig, C, Schmid, I, Conway, E, Schoni, M, Vogt, P, Mueller, C, Fey, M, Zangemeister-Wittke, U & Simon, H-U: Inflammation-associated cell cycle-independent block of apoptosis by survivin in terminally differentiated neutrophils. J Exp Med, 199:1343-54, 2004.
Ambrosini, G, Adida, C & Altieri, D: A novel anti-apoptosis gene, survivin, expressed in cancer and lymphoma. Nature Medicine, 3:917-921, 1997.
Beltrami, E, Plescia, J, Wilkinson, JC, Duckett, CS & Altieri, DC: Acute Ablation of Survivin Uncovers p53-dependent Mitotic Checkpoint Functions and Control of Mitochondrial Apoptosis. J Biol Chem, 279:2077-84, 2004.
Bonegio, R & Lieberthal, W: Role of apoptosis in the pathogenesis of acute renal failure. Curr Opin Nephrol Hypertens, 11:301-8, 2002.
Chen, J, Wu, W, Tahir, SK, Kroeger, PE, Rosenberg, SH, Cowsert, LM, Bennett, F, Krajewski, S, Krajewska, M, Welsh, K, Reed, JC & Ng, SC: Down-regulation of survivin by antisense oligonucleotides increases apoptosis, inhibits cytokinesis and anchorage-independent growth. Neoplasia, 2:235-41, 2000.
Conway, EM, Pollefeyt, S, Cornelissen, J, DeBaere, I, Steiner-Mosonyi, M, Ong, K, Baens, M, Collen, D & Schuh, AC: Three differentially expressed survivin cDNA variants encode proteins with distinct antiapoptotic functions. Blood, 95:1435-42, 2000.
Conway, EM, Pollefeyt, S, Steiner-Mosonyi, M, Luo, W, DeVriese, A, Lupu, F, Bono, F, Leducq, N, Dol, F, Schaeffer, P, Collen, D & Herbert, J-M: Deficiency of Survivin in Transgenic Mice Exacerbates Fas-Induced Apoptosis via Mitochondrial Pathways. Gastroenterology, 123:619.631, 2002.
Conway, EM, Zwerts, F, Van Eygen, V, DeVries, A, Nagai, N, Luo, W & Collen, D: Survivin-dependent angiogenesis in ischemic brain: Molecular mechanisms of hypoxia-induced upregulation. Am J Pathol, 163:935-946, 2003.
Daemen, MA, van 't Veer, C, Denecker, G, Heemskerk, VH, Wolfs, TG, Clauss, M, Vandenabeele, P & Buurman, WA: Inhibition of apoptosis induced by ischemia-reperfusion prevents inflammation. J Clin Invest, 104:541-9, 1999.
Dai, C, Yang, J & Liu, Y: Single injection of naked plasmid encoding hepatocyte growth factor prevents cell death and ameliorates acute renal failure in mice. J Am Soc Nephrol, 13:411-22, 2002.
de Vries, B, Matthijsen, RA, van Bijnen, AA, Wolfs, TG & Buurman, WA: Lysophosphatidic acid prevents renal ischemia-reperfusion injury by inhibition of apoptosis and complement activation. Am J Pathol, 163:47-56, 2003.
Deng, J, Kohda, Y, Chiao, H, Wang, Y, Hu, X, Hewitt, SM, Miyaji, T, McLeroy, P, Nibhanupudy, B, Li, S & Star, RA: Interleukin-10 inhibits ischemic and cisplatin-induced acute renal injury. Kidney Int, 60:2118-28, 2001.
Devarajan, P, Mishra, J, Supavekin, S, Patterson, LT & Steven Potter, S: Gene expression in early ischemic renal injury: clues towards pathogenesis, biomarker discovery, and novel therapeutics. Mol Genet Metab, 80:365-76, 2003.
Evenepoel, P: Acute toxic renal failure. Best Pract Res Clin Anaesthesiol, 18:37-52, 2004.
Fiaschi-Taesch, NM, Santos, S, Reddy, V, Van Why, SK, Philbrick, WF, Ortega, A, Esbrit, P, Orloff, JJ & Garcia-Ocana, A: Prevention of acute ischemic renal failure by targeted delivery of growth factors to the proximal tubule in transgenic mice: the efficacy of parathyroid hormone-related protein and hepatocyte growth factor. J Am Soc Nephrol, 15:112-25, 2004.
Grossman, D, Kim, PJ, Blanc-Brude, OP, Brash, DE, Tognin, S, Marchisio, PC & Altieri, DC: Transgenic expression of survivin in keratinocytes counteracts UVB- induced apoptosis and cooperates with loss of p53. J Clin Invest, 108:991-9., 2001.
Grossman, D, McNiff, JM, Li, F & Altieri, DC: Expression and targeting of the apoptosis inhibitor, survivin, in human melanoma. J Invest Dermatol, 113:1076-81, 1999.
Ito, T, Shiraki, K, Sugimoto, K, Yamanaka, T, Fujikawa, K, Ito, M, Takase, K, Moriyama, M, Kawano, H, Hayashida, M, Nakano, T & Suzuki, A: Survivin Promotes Cell Proliferation in Human Hepatocellular Carcinoma. Hepatology, 31:1080-1085, 2000.
Justo, P, Sanz, A, Lorz, C, Gomez-Garre, D, Mezzano, S, Egido, J & Ortiz, A: Expression of Smac/Diablo in tubular epithelial cells and during acute renal failure. Kidney Int Suppl:S52-6, 2003.
Kaushal, GP: Role of caspases in renal tubular epithelial cell injury. Semin Nephrol, 23:425,31, 2003.
Kelly, KJ, Sutton, TA, Weathered, N, Ray, N, Caldwell, EJ, Plotkin, Z & Dagher, PC: Minocycline inhibits apoptosis and inflammation in a rat model of ischemic renal injury. Am J Physiol Renal Physiol, 2004.
Klingler, EL, Jr., Evan, AP & Anderson, RE: Folic acid-induced renal injury and repair. Correlation of structural and functional abnormalities. Arch Pathol Lab Med, 104:87-93, 1980.
Li, F: Survivin study: what is the next wave? J Cell Physiol, 197:8-29, 2003.
Liu, F, Song, Y & Liu, D: Hydrodynamics-based transfection in animals by systemic administration of plasmid DNA. Gene Ther, 6:1258-66, 1999.
Liu, KD: Molecular mechanisms of recovery from acute renal failure. Crit Care Med, 31:S572-81,2003.
Nogae, S, Miyazaki, M, Kobayashi, N, Saito, T, Abe, K, Saito, H, Nakane, PK, Nakanishi, Y & Koji, T: Induction of apoptosis in ischemia-reperfusion model of mouse kidney: possible involvement of Fas. J Am Soc Nephrol, 9:620-31, 1998.
Oberbauer, R, Rohrmoser, M, Regele, H, Muhlbacher, F & Mayer, G: Apoptosis of tubular epithelial cells in donor kidney biopsies predicts early renal allograft function. J Am Soc Nephrol, 10:2006-13, 1999.
Olie, RA, Simoes-Wust, AP, Baumann, B, Leech, SH, Fabbro, D, Stahel, RA & Zangemeister-Wittke, U: A novel antisense oligonucleotide targeting survivin expression induces apoptosis and sensitizes lung cancer cells to chemotherapy. Cancer Res, 60:2805-9, 2000.
Ortiz, A, Justo, P, Catalan, MP, Sanz, AB, Lorz, C & Egido, J: Apoptotic cell death in renal injury: the rationale for intervention. Curr Drug Targets Immune Endocr Metabol Disord, 2:181-92, 2002.
Ortiz, A, Lorz, C, Catalan, MP, Danoff, TM, Yamasaki, Y, Egido, J & Neilson, EG: Expression of apoptosis regulatory proteins in tubular epithelium stressed in culture or following acute renal failure. Kidney Int, 57:969-81, 2000.
Padanilam, BJ: Cell death induced by acute renal injury: a perspective on the contributions of apoptosis and necrosis. Am J Physiol Renal Physiol, 284:F608-27, 2003.
Sabbatini, M, Pisani, A, Uccello, F, Serio, V, Seru, R, Paterno, R, Cianciaruso, B, Fuiano, G & Andreucci, M: Atorvastatin improves the course of ischemic acute renal failure in aging rats. J Am Soc Nephrol, 15:901-9, 2004.
Schwarz, C, Hauser, P, Steininger, R, Regele, H, Heinze, G, Mayer, G & Oberbauer, R: Failure of BCL-2 up-regulation in proximal tubular epithelial cells of donor kidneys biopsy specimens is associated with apoptosis and delayed graft function. Lab Invest, 82:941-8, 2002.
Sheikh-Hamad, D, Cacini, W, Buckley, AR, Isaac, J, Truong, LD, Tsao, CC & Kishore, BK: Cellular and molecular studies on cisplatin-induced apoptotic cell death in rat kidney. Arch Toxicol, 78:147-55, 2004.
Silvester, W, Bellomo, R & Cole, L: Epidemiology, management, and outcome of severe acute renal failure of critical illness in Australia. Crit Care Med, 29:1910-5,2001.
Tamm, I, Wang, Y, Sausville, E, Scudiero, DA, Vigna, N, Oltersdorf, T & Reed, JC: IAP-family protein survivin inhibits caspase activity and apoptosis induced by Fas (CD95), Bax, caspases, and anticancer drugs. Cancer Res, 58:5315-20, 1998.
Tsuruya, K, Tokumoto, M, Ninomiya, T, Hirakawa, M, Masutani, K, Taniguchi, M, Fukuda, K, Kanai, H, Hirakata, H & lida, M: Antioxidant ameliorates cisplatin-induced renal tubular cell death through inhibition of death receptor-mediated pathways. Am J Physiol Renal Physiol, 285:F208-18, 2003.
Ueda, N & Shah, SV: Tubular cell damage in acute renal failure-apoptosis, necrosis, or both. Nephrol Dial Transplant, 15:318-23, 2000.
Uren, AG, Wong, L, Pakusch, M, Fowler, KJ, Burrows, FJ, Vaux, DL & Choo, KH: Survivin and the inner centromere protein INCENP show similar cell- cycle localization and gene knockout phenotype. Curr Biol, 10:1319-28, 2000.
Wan, L, Bellomo, R, Di Giantomasso, D & Ronco, C: The pathogenesis of septic acute renal failure. Curr Opin Crit Care, 9:496-502, 2003.
Xia, C, Xu, Z, Yuan, X, Uematsu, K, You, L, Li, K, Li, L, McCormick, F & Jablons, DM: Induction of Apoptosis in Mesothelioma Cells by Antisurvivin Oligonucleotides. Mol Cancer Ther, 1:687-694, 2002.

## Claims

1. Use of survivin, or a functional fragment thereof, or a variant thereof having the biological activity of survivin, or a nucleotide sequence encoding survivin, said fragment or said variant, for the manufacture of a medicament to prevent and/or to treat renal failure.

2. The use according to claim 1 wherein said survivin comprises the amino acid sequence depicted in SEQ ID NO: 1.

3. The use according to claim 1 wherein said functional fragment is a fragment that comprises the amino acid sequence as depicted in SEQ ID NO: 3 and that is able to inhibit apoptosis.

4. The use according to claims 1-3 wherein said survivin, functional fragment or variant is fused or chemically coupled to a sequence facilitating protein transduction wherein said sequence is selected from the list comprising HIV TAT protein, penetratin and pep-1.

5. The use according to claim 1 wherein said nucleotide sequence encoding survivin comprises the nucleotide sequence depicted in SEQ ID NO: 1 or wherein said nucleotide sequence encoding a functional fragment comprises the nucleotide sequence depicted in SEQ ID NO:3.

6. The use according to claim 5 wherein said nucleotide sequences are cloned in a vector.

7. The use according to claim 6 wherein said vector is a gene therapy vector.

8. Use according to claims 1-7 wherein said renal failure is chronic or acute renal failure.

9. Use according to claim 8 wherein said acute renal failure is caused by ischemia-reperfusion injury, chemotherapy, kidney transplantation, sepsis and/or heart failure.

10. Survivin or a functional fragment thereof or a variant thereof having the biological activity of survivin, or a nucleotide sequence encoding survivin, said fragment or said variant, for use in preventing and/or to treating renal failure.

## Patentansprüche

1. Verwendung von Survivin oder eines funktionellen Fragments davon oder einer Variante davon mit der biologischen Aktivität von Survivin oder einer Nukleotidsequenz, die Survivin, das Fragment oder die Variante kodiert, zur Herstellung eines Arzneimittels zur Prävention und/oder zur Behandlung von Niereninsuffizienz.

2. Verwendung nach Anspruch 1, wobei das Survivin die in SEQ ID NO: 1 dargestellte Aminosäuresequenz umfasst.

3. Verwendung nach Anspruch 1, wobei es sich bei dem funktionellen Fragment um ein Fragment handelt, das die wie in SEQ ID NO: 3 dargestellte Aminosäuresequenz umfasst und das Apoptose inhibieren kann.

4. Verwendung nach den Ansprüchen 1 - 3, wobei das Survivin, das funktionelle Fragment oder die Variante mit einer Sequenz, die die Proteintransduktion erleichtert, fusioniert oder chemisch gekoppelt wird, wobei die Sequenz aus der Liste ausgewählt ist, die HIV-TAT-Protein, Penetratin und Pep-1 umfasst.

5. Verwendung nach Anspruch 1, wobei die Nukleotidsequenz, die Survivin kodiert, die in SEQ ID NO: 1 dargestellte Nukleotidsequenz umfasst, oder wobei die Nukleotidsequenz, die ein funktionales Fragment kodiert, die in SEQ ID NO: 3 dargestellte Nukleotidsequenz umfasst.

6. Verwendung nach Anspruch 5, wobei die Nukleotidsequenzen in einen Vektor kloniert werden.

7. Verwendung nach Anspruch 6, wobei es sich bei dem Vektor um einen Gentherapievektor handelt.

8. Verwendung nach den Ansprüchen 1 - 7, wobei es sich bei der Niereninsuffizienz um chronische oder akute Niereninsuffizienz handelt.

9. Verwendung nach Anspruch 8, wobei die akute Niereninsuffizienz von Ischämie-Reperfusionsverletzung, Chemotherapie, Nierentransplantation, Sepsis und/oder Herzinsuffizienz verursacht wird.

10. Survivin oder ein funktionelles Fragment davon oder einer Variante davon mit der biologischen Aktivität von Survivin oder eine Nukleotidsequenz, die Survivin, das Fragment oder die Variante kodiert, zur Verwendung in der Prävention und/oder Behandlung von Niereninsuffizienz.

## Revendications

1. Utilisation de survivine, ou d'un fragment fonctionnel de celle-ci, ou d'une variante de celle-ci ayant l'activité biologique de la survivine, ou d'une séquence nucléotidique codant pour la survivine, ledit fragment ou ladite variante, pour la fabrication d'un médicament pour la prévention et/ou le traitement d'une insuffisance rénale.

2. Utilisation selon la revendication 1, dans laquelle ladite survivine comprend la séquence d'acides aminés illustrée par SEQ ID N° : 1.

3. Utilisation selon la revendication 1, dans laquelle ledit fragment fonctionnel est un fragment qui comprend la séquence d'acides aminés telle qu'illustrée par SEQ ID N° : 3 et qui est capable d'inhiber l'apoptose.

4. Utilisation selon les revendications 1 à 3, dans laquelle ladite survivine, ledit fragment fonctionnel ou ladite variante est fusionné(e) ou accouplé(e) chimiquement à une séquence facilitant la transduction protéique dans laquelle ladite séquence est sélectionnée parmi la liste comprenant la protéine TAT du VIH, la pénétratine et pep-1.

5. Utilisation selon la revendication 1, dans laquelle ladite séquence nucléotidique codant pour la survivine comprend la séquence nucléotidique illustrée par SEQ ID N° : 1 ou dans laquelle ladite séquence nucléotidique codant pour un fragment fonctionnel comprend la séquence nucléotidique illustrée par SEQ ID N° : 3.

6. Utilisation selon la revendication 5, dans laquelle lesdites séquences nucléotidiques sont clonées dans un vecteur.

7. Utilisation selon la revendication 6, dans laquelle ledit vecteur est un vecteur de thérapie génique.

8. Utilisation selon les revendications 1 à 7, dans laquelle ladite insuffisance rénale est une insuffisance rénale chronique ou aiguë.

9. Utilisation selon la revendication 8, dans laquelle ladite insuffisance rénale aiguë est provoquée par une ischémie-lésion de reperfusion, une chimiothérapie, une transplantation rénale, une sepsie et/ou une insuffisance cardiaque.

10. Survivine ou fragment fonctionnel de celle-ci ou variante de celle-ci ayant l'activité biologique de la survivine, ou séquence nucléotidique codant pour la survivine, ledit fragment ou ladite variante, destiné(e) à être utilisé(e) dans la prévention et/ou le traitement d'une insuffisance rénale.


### SEQUENCE LISTING

<110> VIB vzw
   D. Collen Research Foundation
<120> USE OF SURVIVIN TO TREAT KIDNEY FAILURE
<130> ECO/Sur/v195
<150> EP 04105744.9
   <151> 2004-11-12
<160> 4
<170> PatentIn version 3.1
<210> 1
   <211> 384
   <212> DNA
   <213> Homo sapiens
<220>
   <221> CDS
   <222> (1) .. (384)
   <223>
<400> 1
   atg ggt gcc ccg acg ttg ccc cct gcc tgg cag ccc ttt ctc aag gac      48
   Met Gly Ala Pro Thr Leu Pro Pro Ala Trp Gln Pro Phe Leu Lys Asp
   1               5                   10                  15
   cac cgc atc tct aca ttc aag aac tgg ccc ttc ttg gag ggc tgc gcc      96
   His Arg Ile Ser Thr Phe Lys Asn Trp Pro Phe Leu Glu Gly Cys Ala
               20                  25                  30
   tgc acc ccg gag cgg atg gcc gag gct ggc ttc atc cac tgc ccc act      144
   Cys Thr Pro Glu Arg Met Ala Glu Ala Gly Phe Ile His Cys Pro Thr
           35                  40                  45
   gag aac gag cca gac ttg gcc cag tgt ttc ttc tgc ttc aag gag ctg      192
   Glu Asn Glu Pro Asp Leu Ala Gln Cys Phe Phe Cys Phe Lys Glu Leu
       50                  55                  60
   gaa ggc tgg gag cca gat gac gac ccc ata gag gaa cat aaa aag cat      240
   Glu Gly Trp Glu Pro Asp Asp Asp Pro Ile Glu Glu His Lys Lys His
   65                  70                  75                  80
   tcg tcc ggt tgc gct ttc ctt tct gtc aag aag cag ttt gaa gaa tta      288
   Ser Ser Gly Cys Ala Phe Leu Ser Val Lys Lys Gln Phe Glu Glu Leu
                   85                  90                  95
   acc ctt ggt gaa ttt ttg aaa ctg gac aga gaa aga gcc aag aac aaa      336
   Thr Leu Gly Glu Phe Leu Lys Leu Asp Arg Glu Arg Ala Lys Asn Lys
               100                 105                 110
   att gta tgt att ggg aat aag aac tgc tca aac cct gtt caa tgt ctt      384
   Ile Val Cys Ile Gly Asn Lys Asn Cys Ser Asn Pro Val Gln Cys Leu
           115                 120                 125
<210> 2
   <211> 128
   <212> PRT
   <213> Homo sapiens
<400> 2
   Met Gly Ala Pro Thr Leu Pro Pro Ala Trp Gln Pro Phe Leu Lys Asp
   1               5                   10                  15
   His Arg Ile Ser Thr Phe Lys Asn Trp Pro Phe Leu Glu Gly Cys Ala
               20                  25                  30
   Cys Thr Pro Glu Arg Met Ala Glu Ala Gly Phe Ile His Cys Pro Thr
           35                  40                  45
   Glu Asn Glu Pro Asp Leu Ala Gln Cys Phe Phe Cys Phe Lys Glu Leu
       50                  55                  60
   Glu Gly Trp Glu Pro Asp Asp Asp Pro Ile Glu Glu His Lys Lys His
   65                  70                  75                  80
   Ser Ser Gly Cys Ala Phe Leu Ser Val Lys Lys Gln Phe Glu Glu Leu
                   85                  90                  95
   Thr Leu Gly Glu Phe Leu Lys Leu Asp Arg Glu Arg Ala Lys Asn Lys
               100                 105                 110
   Ile Val Cys Ile Gly Asn Lys Asn Cys Ser Asn Pro Val Gln Cys Leu
           115                 120                 125
<210> 3
   <211> 231
   <212> DNA
   <213> Homo sapiens
<220>
   <221> CDS
   <222> (1) .. (231)
   <223>
<400> 3
   tgg cag ccc ttt ctc aag gac cac cgc atc tct aca ttc aag aac tgg       48
   Trp Gln Pro Phe Leu Lys Asp His Arg Ile Ser Thr Phe Lys Asn Trp
   1               5                   10                  15
   ccc ttc ttg gag ggc tgc gcc tgc acc ccg gag cgg atg gcc gag gct        96
   Pro Phe Leu Glu Gly Cys Ala Cys Thr Pro Glu Arg Met Ala Glu Ala
               20                  25                  30
   ggc ttc atc cac tgc ccc act gag aac gag cca gac ttg gcc cag tgt       144
   Gly Phe Ile His Cys Pro Thr Glu Asn Glu Pro Asp Leu Ala Gln Cys
           35                  40                  45
   ttc ttc tgc ttc aag gag ctg gaa ggc tgg gag cca gat gac gac ccc       192
   Phe Phe Cys Phe Lys Glu Leu Glu Gly Trp Glu Pro Asp Asp Asp Pro
       50                  55                  60
   ata gag gaa cat aaa aag cat tcg tcc ggt tgc gct ttc                   231
   Ile Glu Glu His Lys Lys His Ser Ser Gly Cys Ala Phe
   65                  70                  75
<210> 4
   <211> 77
   <212> PRT
   <213> Homo sapiens
<400> 4
   Trp Gln Pro Phe Leu Lys Asp His Arg Ile Ser Thr Phe Lys Asn Trp
   1               5                   10                  15
   Pro Phe Leu Glu Gly Cys Ala Cys Thr Pro Glu Arg Met Ala Glu Ala
               20                  25                  30
   Gly Phe Ile His Cys Pro Thr Glu Asn Glu Pro Asp Leu Ala Gln Cys
           35                  40                  45
   Phe Phe Cys Phe Lys Glu Leu Glu Gly Trp Glu Pro Asp Asp Asp Pro
       50                  55                  60
   Ile Glu Glu His Lys Lys His Ser Ser Gly Cys Ala Phe
   65                  70                  75